# EUROPEAN PATENT SPECIFICATION

(11) **EP 4 252 672 B1**
(45) Date of publication and mention of the grant of the patent: **02.07.2025**
(21) Application number: 21897818.7
(22) Date of filing: 17.11.2021
(51) Int. Cl.: A61B 8/14

(54) **INFORMATION PROCESSING DEVICE, ULTRASONIC DIAGNOSIS DEVICE, INFORMATION PROCESSING METHOD, AND INFORMATION PROCESSING PROGRAM**
INFORMATIONSVERARBEITUNGSVORRICHTUNG, ULTRASCHALLDIAGNOSEVORRICHTUNG, INFORMATIONSVERARBEITUNGSVERFAHREN UND INFORMATIONSVERARBEITUNGSPROGRAMM
DISPOSITIF DE TRAITEMENT D'INFORMATIONS, DISPOSITIF DE DIAGNOSTIC PAR ULTRASONS, PROCÉDÉ DE TRAITEMENT D'INFORMATIONS ET PROGRAMME DE TRAITEMENT D'INFORMATIONS

(30) Priority: 27.11.2020 JP 2020197657
(43) Date of publication of application: 04.10.2023
(73) Proprietor: FUJIFILM Corporation, Tokyo 106-8620 (JP)
(72) Inventor: MATSUMOTO, Tsuyoshi, Tokyo 106-8620 (JP)
(74) Representative: Dehns Germany Partnerschaft mbB
(86) International application number: PCT/JP2021/042220
(87) International publication number: WO 2022/113844

(56) References cited:
- JP-A- 2004 141 346
- JP-A- 2004 141 346
- JP-A- 2009 045 427
- JP-A- 2009 045 427
- JP-A- H1 133 026
- JP-A- H11 128 224
- US-A1- 2016 133 028

## Description

### Technical Field

The present disclosure relates to an information processing apparatus, an ultrasound diagnostic apparatus, an information processing method, and an information processing program.

### Background Art

An ultrasound diagnostic apparatus that captures an ultrasound image of a subject using an ultrasound probe that receives an ultrasound echo by ultrasound transmitted toward the subject and outputs a reception signal based on the received ultrasound echo is known.

In addition, a blood vessel of the subject is punctured by inserting an insert, such as a so-called puncture needle or a catheter. As a puncture method, such as an echo-guided puncture method, a method is known in which the ultrasound image of the blood vessel of the subject is captured and a puncture is performed with reference to the blood vessel shown in the captured ultrasound image. In the present method, there is technology of presenting information indicating a degree of difficulty of the puncture to a user, such as a doctor or a nurse who performs the puncture. For example, JP2016-123794A discloses technology of comparing a blood vessel diameter detected from an ultrasound image with a standard blood vessel diameter, determining whether the puncture is possible or impossible from a collapsed state of the blood vessel using an ultrasound probe, and presenting whether the puncture is possible or impossible. Another prior art example is the publication JP2004-141346A disclosing an ultrasonic evaluation device comprising an evaluation unit for outputting a puncture difficulty level.

### SUMMARY OF THE INVENTION

### Technical Problem

By the way, a skill level of the puncture differs according to the user. Therefore, the ease of the puncture may differ according to an individual user. In JP2016-123794A, whether the puncture is possible or impossible is presented as the degree of difficulty of the puncture, but the presented degree of difficulty of the puncture does not correspond to the individual user, and may not be appropriate for the user who actually performs the puncture.

The present disclosure has been made in view of the above circumstances, and is to provide an information processing apparatus, an ultrasound diagnostic apparatus, an information processing method, and an information processing program capable of presenting information indicating an appropriate degree of difficulty of the puncture according to the user who performs the puncture of the blood vessel.

### Solution to Problem

In order to achieve the above object, a first aspect of the present disclosure relates to an information processing apparatus comprising an acquisition unit that acquires an ultrasound image of a tissue including a blood vessel of a subject, a detection unit that detects the blood vessel from the acquired ultrasound image, and a degree-of-difficulty information output unit that outputs degree-of-difficulty information related to a degree of difficulty of a puncture in the detected blood vessel according to a skill level of a user who performs the puncture.

A second aspect of the present disclosure relates to the information processing apparatus according to the first aspect, further comprising a determination unit that determines whether the puncture is possible or impossible based on a detection result of the detection unit, in which the degree-of-difficulty information output unit uses a determination result of the determination unit as the degree-of-difficulty information.

A third aspect of the present disclosure relates to the information processing apparatus according to the second aspect, in which it is determined whether the puncture is possible or impossible based on a blood vessel condition related to whether the puncture is possible or impossible according to the skill level of the user, and the detection result.

A fourth aspect of the present disclosure relates to the information processing apparatus according to the second or third aspect, in which, in a case in which an image quality of the ultrasound image is higher than a predetermined image quality, the determination unit relaxes the blood vessel condition in which the puncture is possible.

A fifth aspect of the present disclosure relates to the information processing apparatus according to any one of the second to fourth aspects, in which the detection unit further detects a depth from a body surface of the subject to the blood vessel, and the determination unit determines whether the puncture is possible or impossible based on the depth.

A sixth aspect of the present disclosure relates to the information processing apparatus according to the fifth aspect, in which, in a case in which the depth is smaller than a depth threshold value, the determination unit determines that the puncture is possible.

A seventh aspect of the present disclosure relates to the information processing apparatus according to any one of the second to sixth aspects, in which the detection unit further detects a blood vessel diameter indicating a thickness of the blood vessel, and the determination unit determines whether the puncture is possible or impossible based on the blood vessel diameter.

An eighth aspect of the present disclosure relates to the information processing apparatus according to the seventh aspect, in which, in a case in which the blood vessel diameter is larger than a blood vessel diameter threshold value, the determination unit determines that the puncture is possible.

A ninth aspect of the present disclosure relates to the information processing apparatus according to any one of the second to eighth aspects, in which the detection unit further detects a tissue surrounding the blood vessel, and the determination unit determines whether the puncture is possible or impossible based on the tissue.

A tenth aspect of the present disclosure relates to the information processing apparatus according to the ninth aspect, in which, in a case in which a distance between the blood vessel and the tissue is equal to or larger than a spacing threshold value, the determination unit determines that the puncture is possible.

An eleventh aspect of the present disclosure relates to the information processing apparatus according to the ninth or tenth aspect, in which the blood vessel is a target blood vessel that is a target for the puncture, and the tissue includes a non-target blood vessel that is not the target for the puncture.

A twelfth aspect of the present disclosure relates to the information processing apparatus according to any one of the first to eleventh aspects, in which the degree-of-difficulty information is information of which a display form differs depending on whether the puncture is possible or impossible.

A thirteenth aspect of the present disclosure relates to the information processing apparatus according to any one of the second to twelfth aspects, in which, in a case in which an image quality of the ultrasound image is higher than a predetermined reference, the determination unit increases the skill level, which is acquired, and determines whether the puncture is possible or impossible based on a blood vessel condition according to the increased skill level.

A fourteenth aspect of the present disclosure relates to the information processing apparatus according to any one of the first to thirteenth aspects, in which the degree-of-difficulty information output unit outputs the ultrasound image to which the degree-of-difficulty information is added.

In addition, in order to achieve the above object, a fifteenth aspect of the present disclosure relates to an ultrasound diagnostic apparatus comprising an ultrasound probe that receives an ultrasound echo from transmitted ultrasound, and outputs a reception signal based on the received ultrasound echo, an image generation unit that generates an ultrasound image based on the reception signal input from the ultrasound probe, and the information processing apparatus according to the present disclosure.

In addition, in order to achieve the above object, a sixteenth aspect of the present disclosure relates to an information processing method executed by a computer, the method comprising acquiring an ultrasound image of a tissue including a blood vessel of a subject, detecting the blood vessel from the acquired ultrasound image, and outputting degree-of-difficulty information related to a degree of difficulty of a puncture in the detected blood vessel according to a skill level of a user who performs the puncture.

In addition, in order to achieve the above object, a seventeenth aspect of the present disclosure relates to an information processing program causing a computer to execute a process comprising acquiring an ultrasound image of a tissue including a blood vessel of a subject, detecting the blood vessel from the acquired ultrasound image, and outputting degree-of-difficulty information related to a degree of difficulty of a puncture in the detected blood vessel according to a skill level of a user who performs the puncture.

### Advantageous Effects of Invention

According to the present disclosure, it is possible to present the information indicating an appropriate degree of difficulty of the puncture according to the user who performs the puncture of the blood vessel.

### BRIEF DESCRIPTION OF THE DRAWINGS

Fig. 1 is a block diagram showing an example of an overall configuration of an ultrasound diagnostic apparatus according to an embodiment.
Fig. 2 is a block diagram showing an example of a configuration of a reception circuit.
Fig. 3 is a block diagram showing an example of a configuration of an image generation unit.
Fig. 4A is a diagram showing an example of an ultrasound image captured by a minor axis method.
Fig. 4B is a diagram showing an example of an ultrasound image captured by a major axis method.
Fig. 4C is a diagram for describing a blood vessel diameter and a depth of a blood vessel.
Fig. 5A is a diagram for describing an example of personal skill level information.
Fig. 5B is a diagram for describing an example of blood vessel condition information.
Fig. 6 is a configuration diagram showing an example of a hardware configuration of a body part according to the embodiment.
Fig. 7 is a flowchart showing an example of a flow of puncture assist processing by the body part according to the embodiment.
Fig. 8A is a diagram showing an example of an ultrasound image to which degree-of-difficulty information indicating that a puncture is possible is added.
Fig. 8B is a diagram showing an example of an ultrasound image to which degree-of-difficulty information indicating that the puncture is impossible is added.
Fig. 9 is a diagram for describing another example of the blood vessel condition information.
Fig. 10 is a diagram for describing an example of a blood vessel condition according to a modification example 1.
Fig. 11 is a flowchart showing an example of a flow of puncture assist processing by a body part according to the modification example 1.
Fig. 12A is a diagram showing an example of an ultrasound image to which degree-of-difficulty information indicating that a puncture is possible in the modification example is added.
Fig. 12B is a diagram showing an example of an ultrasound image to which degree-of-difficulty information indicating that the puncture is impossible in the modification example is added.
Fig. 13 is a diagram showing an example of an overall configuration of a modification example of the ultrasound diagnostic apparatus.
Fig. 14 is a diagram showing an example of an overall configuration of another modification example of the ultrasound diagnostic apparatus.

### DESCRIPTION OF EMBODIMENTS

Hereinafter, description of an embodiment of the present invention will be made in detail with reference to the drawings. It should be noted that the present embodiment does not limit the present invention.

### [First Embodiment]

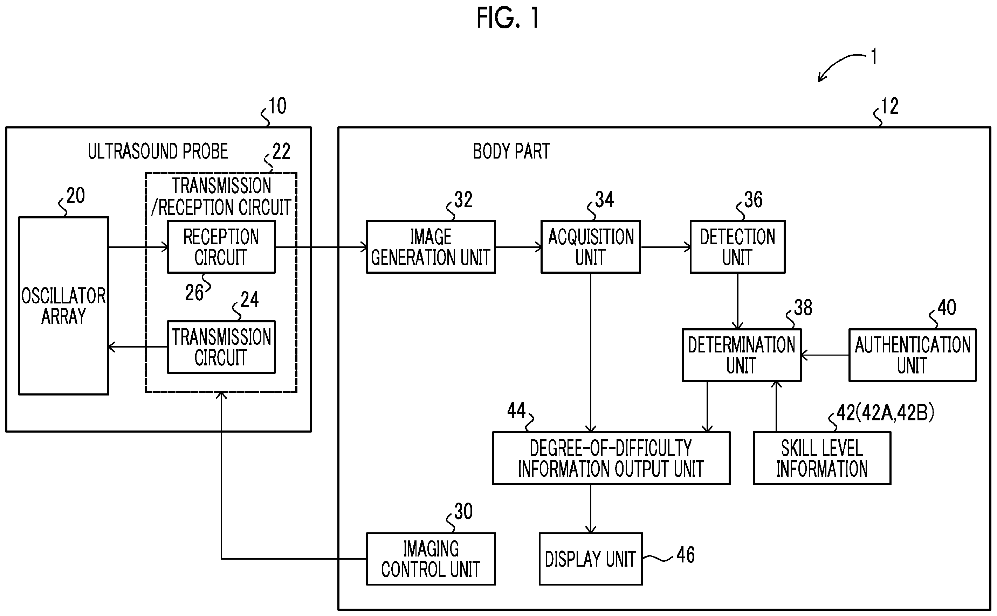

First, an example of an overall configuration of an ultrasound diagnostic apparatus according to the present embodiment will be described. Fig. 1 shows a block diagram showing an example of an overall configuration of an ultrasound diagnostic apparatus 1 according to the present embodiment. As shown in Fig. 1, the ultrasound diagnostic apparatus 1 according to the present embodiment comprises an ultrasound probe 10 and a body part 12.

The ultrasound probe 10 comprises an oscillator array 20 and a transmission/reception circuit 22 including a transmission circuit 24 and a reception circuit 26. The oscillator array 20 comprises a plurality of oscillators (not shown) arranged in a one-dimensional or two-dimensional manner. As an example, in the present embodiment, a form will be described in which the ultrasound probe 10 is a linear-type ultrasound probe in which the plurality of oscillators are linearly arranged. It should be noted that the ultrasound probe 10 is not limited to the present form, and may be a convex-type or sector-type ultrasound probe in which oscillators are arranged in a curved manner. Each of the plurality of oscillators transmits ultrasound based on a drive signal applied from the transmission circuit 24, receives an ultrasound echo generated in a subject, and outputs an electrical signal according to the received ultrasound echo.

Each of the plurality of oscillators is configured by, for example, forming electrodes on both ends of a piezoelectric body which is a material having piezoelectricity, such as a piezoelectric ceramic typified by Lead Zirconate Titanate (PZT), polymer piezoelectric element represented by Poly Vinylidene Di Fluoride (PVDF), or a piezoelectric single crystal represented by Lead Magnesium Niobate-Lead Titanate (PMN-PT).

The transmission circuit 24 causes the oscillator array 20 to transmit an ultrasound beam toward the subject. Specifically, the transmission circuit 24 includes, for example, a plurality of pulse generators (not shown), and adjusts, based on a transmission delay pattern selected according to a control signal from an imaging control unit 30 of the body part 12, a delay amount of each of the plurality of oscillators of the oscillator array 20 and supplies the drive signal to apply a voltage. Each of the drive signals is a pulse-like or continuous wave-like voltage signal, and the piezoelectric body expands and contracts in a case in which the voltage is applied to the electrodes of the oscillators of the oscillator array 20. As a result of the above, pulse-like or continuous wave-like ultrasound is generated from each of the oscillators, and the ultrasound beam is formed from the ultrasound combination wave.

The transmitted ultrasound beam is reflected by each part (for example, a blood vessel or another tissue) in the subject, an instrument disposed in the subject, or the like to generate the ultrasound echo. The generated ultrasound echo propagates in the subject and is received by the plurality of oscillators of the oscillator array 20. Each oscillator generates the electrical signal according to the received ultrasound echo. The electrical signal generated in each oscillator is output to the reception circuit 26.

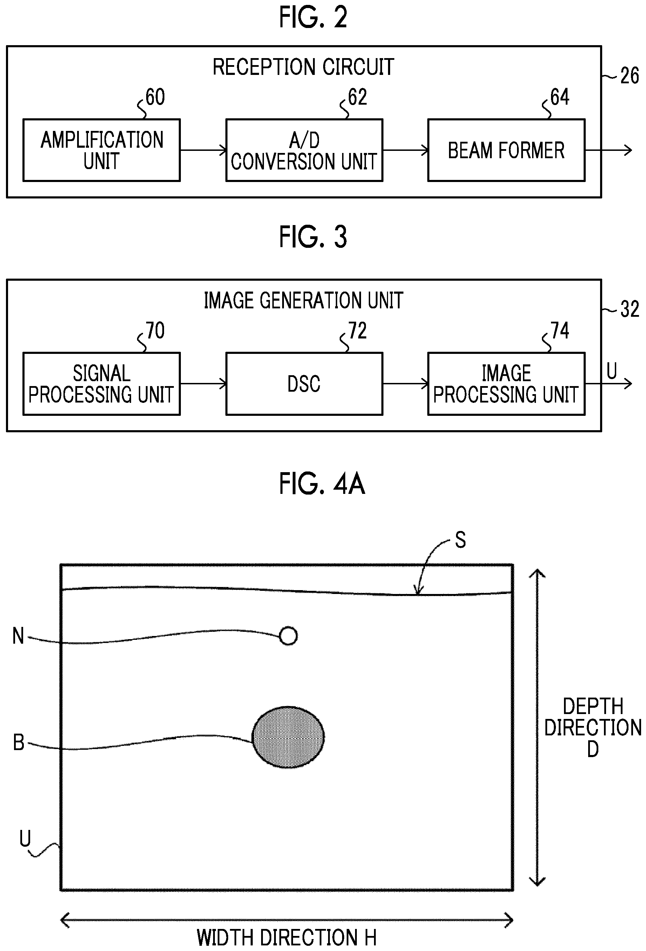

The reception circuit 26 generates a sound ray signal by performing processing on a signal (strictly speaking, an analog electrical signal) output from the oscillator array 20 according to the control signal from the imaging control unit 30 of the body part 12. Fig. 2 shows a block diagram showing an example of a configuration of the reception circuit 26 according to the present embodiment. As shown in Fig. 2, the reception circuit 26 includes, for example, an amplification unit 60, an analog digital (A/D) conversion unit 62, and a beam former 64.

The amplification unit 60 amplifies the electrical signal output from each of the plurality of oscillators of the oscillator array 20, and outputs the amplified electrical signal to the AD conversion unit 62. The AD conversion unit 62 converts the amplified electrical signal into digital reception data to output each of the converted reception data to the beam former 64. The beam former 64 performs reception focus processing by giving and adding the delay to each reception data converted by the AD conversion unit 62 according to a sound velocity or a sound velocity distribution set based on a reception delay pattern selected according to the control signal from the imaging control unit 30 of the body part 12. In the reception focus processing, each reception data converted by the AD conversion unit 62 is phase-adjusted and added, and the sound ray signal in which the focus of the ultrasound echo is narrowed down is generated. The generated sound ray signal is output to the image generation unit 32 of the body part 12.

On the other hand, the body part 12 comprises an imaging control unit 30, the image generation unit 32, an acquisition unit 34, a detection unit 36, a determination unit 38, an authentication unit 40, a degree-of-difficulty information output unit 44, and a display unit 46. As an example, the body part 12 according to the present embodiment is a portable terminal device, such as a smartphone or a tablet terminal. The body part 12 has a function of capturing the ultrasound image which is a B-mode image (tomographic image) related to the tissue in the subject by the sound ray signal obtained by scanning the subject with the ultrasound probe 10 by installing a program, such as application software. The body part 12 according to the present embodiment is an example of an information processing apparatus according to the present disclosure.

The imaging control unit 30 has a function of outputting the control signal to the transmission/reception circuit 22 of the ultrasound probe 10 as described above in a case in which the ultrasound image is captured. By inputting the control signal output from the imaging control unit 30 to the transmission circuit 24 and the reception circuit 26, the sound ray signal is output from the reception circuit 26 of the ultrasound probe 10 to the image generation unit 32, as described above.

The image generation unit 32 has a function of generating the ultrasound image based on the sound ray signal input from the reception circuit 26 of the ultrasound probe 10. Fig. 3 shows a block diagram showing an example of a configuration of the image generation unit 32 according to the present embodiment. As shown in Fig. 3, the image generation unit 32 includes, for example, a signal processing unit 70, a digital scan converter (DSC) 72, and an image processing unit 74. The signal processing unit 70 corrects the attenuation by the distance according to the depth of the reflection position of the ultrasound for the sound ray signal generated by the reception circuit 26, and then performs envelope detection processing to generate a B-mode image signal indicating an ultrasound image U. The DSC 72 converts the B-mode image signal generated by the signal processing unit 70 into an image signal according to a normal television signal scanning method, by raster conversion or the like. The image processing unit 74 performs various necessary image processing, such as gradation processing, on the B-mode image signal input from the DSC 72, and then outputs the B-mode image signal. The B-mode image signal output from the image generation unit 32 corresponds to the ultrasound image U.

Under the control of the imaging control unit 30, the transmission/reception circuit 22 of the ultrasound probe 10 and the image generation unit 32 of the body part 12 continuously acquire the ultrasound images at a certain frame rate a plurality of times during an imaging period of the ultrasound image.

It should be noted that a part at which the tomography is observed is changed depending on the ultrasound image by moving the ultrasound probe 10 in a state of being in contact with the subject, and an observation direction of the blood vessel or the like in the subject can be switched by changing a direction in which the ultrasound probe 10 is brought into contact with the subject. For example, in a case in which the ultrasound probe 10 is brought into contact with the subject in a direction intersecting an extension direction of the blood vessel and an insert in a direction in which the plurality of oscillators are arranged (that is, a scanning direction) in the oscillator array 20, that is, in a case in which the minor axis method (intersection method) is adopted, transverse cross sections of the blood vessel and the insert are observed in the ultrasound image. Fig. 4A shows an example of the ultrasound image U captured by a minor axis method. The ultrasound image U shown in Fig. 4A shows a transverse cross section of a blood vessel B and a transverse cross section of a puncture needle N, which is an example of an insert. The transverse cross section of each of the blood vessel B and the puncture needle N here means a cut surface orthogonal to the extension direction of each of the blood vessel B and the puncture needle N.

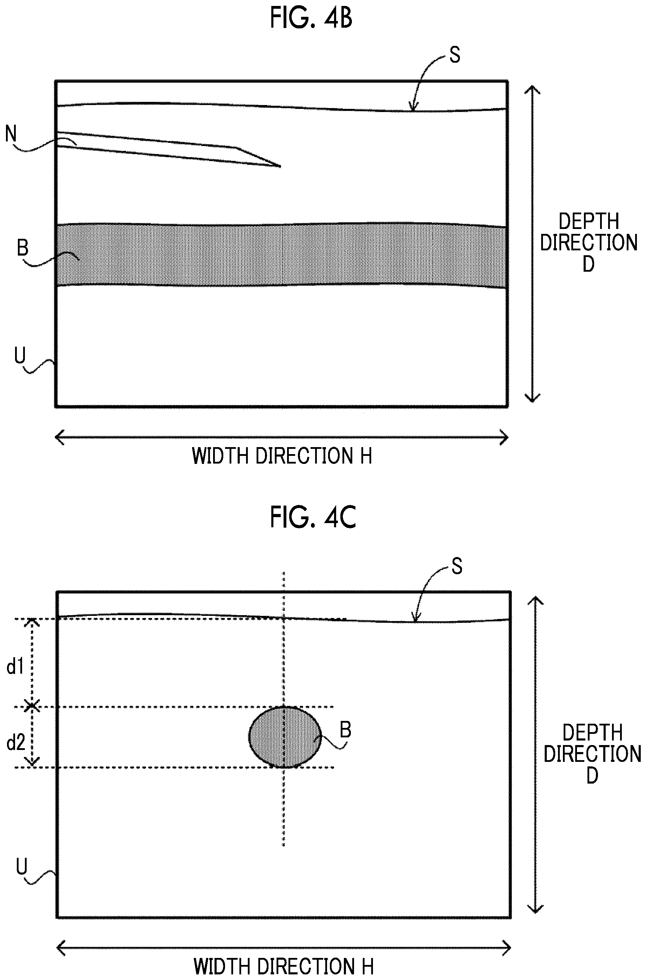

On the other hand, in a case in which the ultrasound probe 10 is brought into contact with the subject in a direction in which an arrangement direction (scanning direction) of the oscillators in the oscillator array 20 is along the extension direction of the blood vessel and the insert, that is, in a case in which a major axis method (paralleling method) is adopted, longitudinal cross sections of the blood vessel and the insert are observed in the ultrasound image. Fig. 4B shows an example of the ultrasound image U captured by a major axis method. The ultrasound image U shown in Fig. 4B shows a longitudinal cross section of the blood vessel B and a longitudinal cross section of the puncture needle N, which is the example of the insert. The longitudinal cross section of each of the blood vessel B and the puncture needle N here means a cut surface along the extension direction of each of the blood vessel B and the puncture needle N.

It should be noted that, in the present embodiment, as shown in Figs. 4A and 4B, a direction connecting a body surface S and an inside of the subject in the ultrasound image U is referred to as a depth direction D. The depth direction D corresponds to a direction in which a plurality of scanning lines extend in the ultrasound image U. On the other hand, a direction intersecting the depth direction D is referred to as a width direction H. The width direction H corresponds to a direction in which the plurality of scanning lines are arranged. Each portion of the ultrasound image U, such as the blood vessel B or the puncture needle N, is displayed at the position according to the distance from the body surface of the subject with which the ultrasound probe 10 is brought into contact in the depth direction D, that is, the depth.

The ultrasound image U generated by the image generation unit 32 is output to the acquisition unit 34.

The acquisition unit 34 has a function of acquiring the ultrasound image U generated and output by the image generation unit 32. The ultrasound image U acquired by the acquisition unit 34 is output to the detection unit 36 and the degree-of-difficulty information output unit 44. It should be noted that, in a case in which the image generation unit 32 functions as the information processing apparatus according to the present disclosure as in the present embodiment, the functions of the image generation unit 32 and the acquisition unit 34 may be integrated. In other words, in a case in which the information processing apparatus according to the present disclosure comprises the image generation unit 32, the image generation unit 32 may further function as the acquisition unit 34.

The detection unit 36 has a function of detecting the blood vessel B from the ultrasound image U input from the acquisition unit 34. It should be noted that the method in which the detection unit 36 detects the blood vessel B from the ultrasound image U is not particularly limited. As an example, the detection unit 36 according to the present embodiment analyzes the ultrasound image U acquired by the acquisition unit 34, in other words, the ultrasound image U generated by the image generation unit 32, according to a known algorithm, to detect the blood vessel B in the ultrasound image U. For example, the detection unit 36 can store typical pattern data of a blood vessel region as a template in advance, derive a degree of similarity with respect to the pattern data while searching the ultrasound image U with the template, and consider that the blood vessel B is present in a place in which the degree of similarity is equal to or larger than a reference value and is maximized.

Also, in addition to simple template matching, the derivation of the degree of similarity includes a method using a trained learning model based on a feature amount of an image showing the blood vessel B. For example, a machine learning method, such as Support Vector Machine (SVM) or Adaptive Boosting (AdaBoost) described in Csurka et al.: Visual Categorization with Bags of Keypoints, Proc. of ECCV Workshop on Statistical Learning in Computer Vision, pp. 59-74 (2004), or a general image recognition method using deep learning described in Krizhevsk et al.: ImageNet Classification with Deep Convolutional Neural Networks, Advances in Neural Information Processing Systems 25, pp. 1106-1114 (2012) can be used.

In addition, for example, the detection unit 36 may detect the blood vessel B in the ultrasound image U using a blood vessel detection model which is a trained model that has been subjected to machine learning using a plurality of ultrasound images U labeled with respect to the blood vessel B. For example, the blood vessel detection model is an object detection algorithm using deep learning. As the blood vessel detection model, for example, an object detection model configured by a regional convolutional neural network (R-CNN), which is a type of a CNN, can be used. The blood vessel detection model detects the blood vessel B as an object from the input ultrasound image U, and outputs information indicating the blood vessel B in the ultrasound image U.

Further, the detection unit 36 according to the present embodiment has a function of detecting a diameter (hereinafter, referred to as a "blood vessel diameter") and a depth of the detected blood vessel B. It should be noted that the method in which the detection unit 36 derives each of the blood vessel diameter and the depth of the blood vessel B in the ultrasound image U is not particularly limited. As an example, the detection unit 36 according to the present embodiment applies known image analysis processing to the blood vessel B in the detected ultrasound image U to derive the blood vessel diameter and the depth of the blood vessel B. It should be noted that, as shown in Fig. 4C, the blood vessel diameter of the blood vessel B means a width (length indicated by a reference numeral d2 in Fig. 4C) of the blood vessel B in the depth direction D of the ultrasound image U. In addition, the depth of the blood vessel B means the shortest distance (distance indicated by a reference numeral d1 in Fig. 4C) from the position corresponding to the body surface S of the subject in the ultrasound image U to the blood vessel B in the depth direction D.

It should be noted that the detection unit 36 according to the present embodiment detects all the blood vessels B included in the ultrasound image U, and the depth and the blood vessel diameter thereof. In a case in which a plurality of blood vessels B are included in the ultrasound image U, a form may be adopted in which the detection is performed for each of the plurality of blood vessels B by applying, for example, the template matching, the general image recognition method, or the blood vessel detection model. In addition, for example, a form may be adopted in which one or more blood vessels B are collectively detected by applying the template matching, the general image recognition method, or the blood vessel detection model.

A detection result of the detection unit 36 is output to the determination unit 38. Specifically, the information indicating the blood vessel B in the ultrasound image U detected by the detection unit 36, and the depth and the blood vessel diameter of the blood vessel B are output to the determination unit 38.

The determination unit 38 has a function of determining whether a puncture is possible or impossible based on blood vessel information according to a skill level of a user and the detection result of the detection unit 36. Specifically, the determination unit 38 determines whether the puncture is possible or impossible for the blood vessel B based on a blood vessel condition in which the puncture is possible according to the skill level of the user for the blood vessel B in the ultrasound image U, and the depth and the blood vessel diameter of the blood vessel B. It should be noted that the skill level is a skill level for the puncture of the blood vessel B of the user, such as a doctor or a nurse who performs the puncture, and in the present embodiment, the skill level is higher as the user is better at the puncture, and the skill level is lower as the user is worse at the puncture or is the beginner for the puncture.

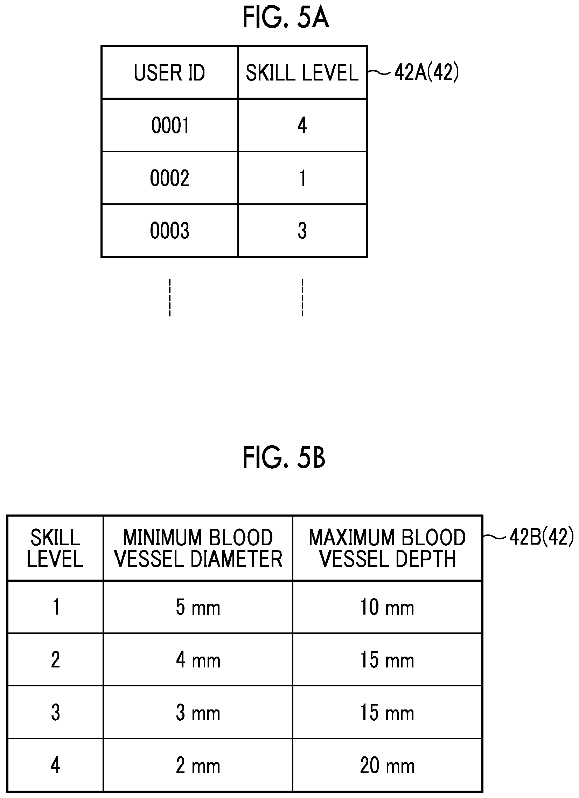

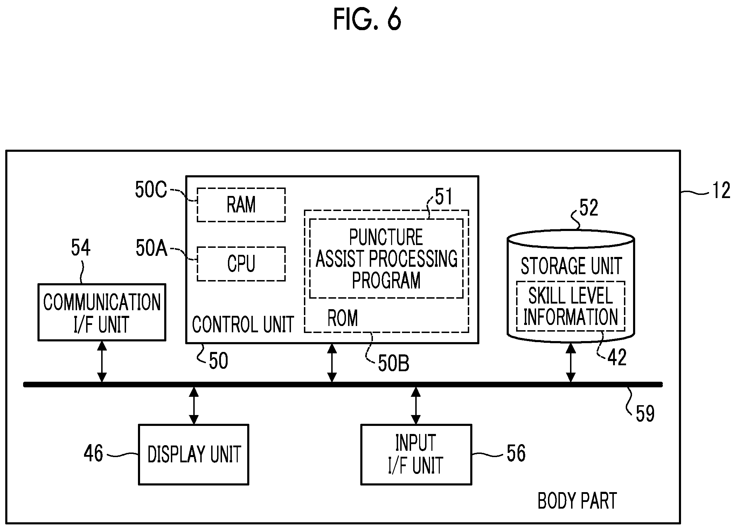

A storage unit 52 (see Fig. 6) according to the present embodiment stores skill level information 42 indicating the blood vessel condition in which the puncture is permitted according to the skill level for each user. It should be noted that the blood vessel condition is a condition indicating a state of the blood vessel B as to whether the puncture is possible or impossible, and in the present embodiment, the minimum blood vessel diameter and the maximum blood vessel depth at which the puncture is possible are adopted. As an example, the skill level information 42 according to the present embodiment includes personal skill level information 42A, which is shown as an example in Fig. 5A, and blood vessel condition information 42B, which is shown as an example in Fig. 5B.

The personal skill level information 42A is information indicating a correspondence relationship between a user identification (ID) for identifying the user and the skill level of the user. As a specific example, in the present embodiment, four stages from "1" to "4" are provided for the skill level, and the skill is higher as the number of stages is larger. That is, a case in which the skill level is "4" indicates that the skill is highest, and a case in which the skill level is "1" indicates that the skill is lowest. The personal skill level information 42A is registered in advance for each user who uses the ultrasound diagnostic apparatus 1. It should be noted that the method of setting the skill level of each user is not particularly limited, and for example, the setting may be possible by an administrator of the ultrasound diagnostic apparatus 1.

The blood vessel condition information 42B is information indicating the correspondence relationship between the skill level and the minimum blood vessel diameter and the maximum blood vessel depth at which the puncture is possible. One factor that determines a degree of difficulty of the puncture of the blood vessel B is the blood vessel diameter of the blood vessel B. In general, as the blood vessel diameter of the blood vessel B is smaller, it is more difficult to perform the puncture, and the difficulty of the puncture is higher. On the other hand, as the blood vessel diameter of the blood vessel B is larger, it is easier to perform the puncture, and the degree of difficulty of the puncture is lower, in other words, a degree of easiness of the puncture is higher. Therefore, in the present embodiment, the minimum blood vessel diameter at which the puncture is possible is determined for each skill level. The minimum blood vessel diameter in the blood vessel condition information 42B according to the present embodiment is an example of a blood vessel diameter threshold value according to the present disclosure.

In addition, the depth of the blood vessel B is described as another factor that determines the degree of difficulty of the puncture of the blood vessel B. In general, as the depth of the blood vessel B is larger, it is more difficult to perform the puncture, and the degree of difficulty of the puncture is higher. On the other hand, as the depth of the blood vessel B is smaller, it is easier to perform the puncture, and the degree of difficulty of the puncture is lower, in other words, the degree of easiness of the puncture is higher. Therefore, in the present embodiment, the maximum blood vessel depth at which the puncture is possible is determined for each skill level. The maximum blood vessel depth in the blood vessel condition information 42B according to the present embodiment is an example of a depth threshold value according to the present disclosure.

Specifically, the determination unit 38 specifies the skill level corresponding to the user ID input from the authentication unit 40 with reference to the personal skill level information 42A. In addition, the determination unit 38 derives the minimum blood vessel diameter and the maximum blood vessel depth, which correspond to the specified skill level, with reference to the blood vessel condition information 42B. Further, the determination unit 38 determines whether the puncture is possible or impossible by comparing each of the derived minimum blood vessel diameter and maximum blood vessel depth with the blood vessel diameter and the depth of the blood vessel B which are the detection results input from the detection unit 36. For example, in a case in which the user ID input from the authentication unit 40 is "0003", first, the determination unit 38 specifies that the skill level is "3" with reference to the personal skill level information 42A. Also, the determination unit 38 derives "3 mm" for the minimum blood vessel diameter corresponding to the skill level of "3" and "15 mm" for the maximum blood vessel depth with reference to the blood vessel condition information 42B. Moreover, in a case in which the blood vessel diameter input from the detection unit 36 is equal to or larger than "3 mm" and the depth is equal to or smaller than "15 mm", the determination unit 38 determines that the puncture is possible. In other words, the determination unit 38 determines that the puncture is impossible in at least one of a case in which the blood vessel diameter input from the detection unit 36 is smaller than "3 mm" or a case in which the depth is larger than "15 mm".

The determination unit 38 outputs, as the determination result, information indicating whether the puncture is possible or impossible to the degree-of-difficulty information output unit 44.

The authentication unit 40 has a function of performing authentication processing for the user who uses the ultrasound diagnostic apparatus 1. Specifically, the authentication unit 40 has a function of acquiring the user ID of the user who uses the ultrasound diagnostic apparatus 1. It should be noted that the method in which the authentication unit 40 authenticates the user is not particularly limited. For example, a form may be adopted in which the authentication unit 40 acquires the user ID input by the user or the like by operating an input interface (I/F) unit 56 (see Fig. 6) provided in the body part 12. In addition, for example, in a case in which user IDs of a plurality of users who use the ultrasound diagnostic apparatus 1 are registered in advance, a form may be adopted in which the plurality of registered user IDs are displayed on the display unit 46, and the authentication unit 40 acquires the user ID selected by the user through the input I/F unit 56 from among the plurality of registered user IDs. In addition, for example, in a case in which a correspondence relationship between the user IDs and biometric information of the user, such as a fingerprint, a voice print, an iris, and a face, is registered in advance for the plurality of users who use the ultrasound diagnostic apparatus 1, a form may be adopted in which the authentication unit 40 acquires the user ID based on the biometric information of the user read through the input I/F unit 56 or the like and the registered correspondence relationship. In addition, for example, a form may be adopted in which the authentication unit 40 acquires the user ID by reading the user ID, such as an employee ID card owned by the user, through the input I/F unit 56 or the like.

The user ID acquired by the authentication unit 40 is output to the determination unit 38.

The degree-of-difficulty information output unit 44 has a function of outputting degree-of-difficulty information related to the degree of difficulty of the puncture in the blood vessel B detected by the detection unit 36 according to the skill level of the user who performs the puncture. Specifically, the degree-of-difficulty information output unit 44 according to the present embodiment adds, as the degree-of-difficulty information, information indicating whether the puncture is possible or impossible, which is a determination result of the determination unit 38, to the ultrasound image U input from the acquisition unit 34, and outputs the information to the display unit 46.

The display unit 46 has a function of displaying various types of information, such as the ultrasound image U to which the degree-of-difficulty information output from the degree-of-difficulty information output unit 44, that is, the information indicating whether the puncture is possible or impossible is added. Examples of the display unit 46 include a liquid crystal display (LCD), an organic electro luminescence (EL) display, and a head mounted display.

The body part 12 can be configured by, for example, the hardware shown in Fig. 6. Fig. 6 shows a configuration diagram showing an example of a hardware configuration of the body part 12 according to the present embodiment. As shown in Fig. 6, the body part 12 comprises the display unit 46, a control unit 50, the storage unit 52, a communication interface (I/F) unit 54, and the input I/F unit 56. The display unit 46, the control unit 50, the storage unit 52, the communication I/F unit 54, and the input I/F unit 56 are connected to each other through a bus 59, such as a system bus or a control bus, such that various types of information can be exchanged.

The control unit 50 controls an overall operation of the body part 12. The control unit 50 comprises a central processing unit (CPU) 50A, a read only memory (ROM) 50B, and a random access memory (RAM) 50C. The ROM 50B stores, in advance, various programs and the like, which are executed by the CPU 50A and include a puncture assist processing program 51 and an imaging program (not shown). The RAM 50C transitorily stores various data. The puncture assist processing program 51 according to the present embodiment is an example of an information processing program according to the present disclosure.

The CPU 50A executes the imaging program stored in the ROM 50B, so that the CPU 50A functions as the imaging control unit 30. In addition, the CPU 50A executes the puncture assist processing program 51 stored in the ROM 50B, so that the CPU 50A functions as the acquisition unit 34, the detection unit 36, the determination unit 38, the authentication unit 40, the skill level information 42, and the degree-of-difficulty information output unit 44.

The storage unit 52 stores image data of the ultrasound image U generated by the image generation unit 32, the skill level information 42, various other information, and the like. Specific examples of the storage unit 52 include a hard disk drive (HDD), a solid state drive (SSD), and a secure digital (SD) card.

The input I/F unit 56 is used for the user to input instructions and various types of information related to capturing of the ultrasound image U and the like. The input I/F unit 56 is not particularly limited, and examples thereof include various switches, a touch panel, a touch pen, a camera, and a mouse. It should be noted that the display unit 46 and the input I/F unit 56 may be integrated to form a touch panel display.

The communication I/F unit 54 performs communication of various types of information with the ultrasound probe 10 or an external device of the ultrasound diagnostic apparatus 1 by wireless communication, such as WiFi (registered trademark) or Bluetooth (registered trademark) or wired communication. As described above, the body part 12 outputs the control signal for capturing the ultrasound image U to the ultrasound probe 10 through the communication I/F unit 54. In addition, the sound ray signal is input from the ultrasound probe 10 to the body part 12 through the communication I/F unit 54.

Next, an action of the body part 12 according to the present embodiment will be described with reference to the drawings.

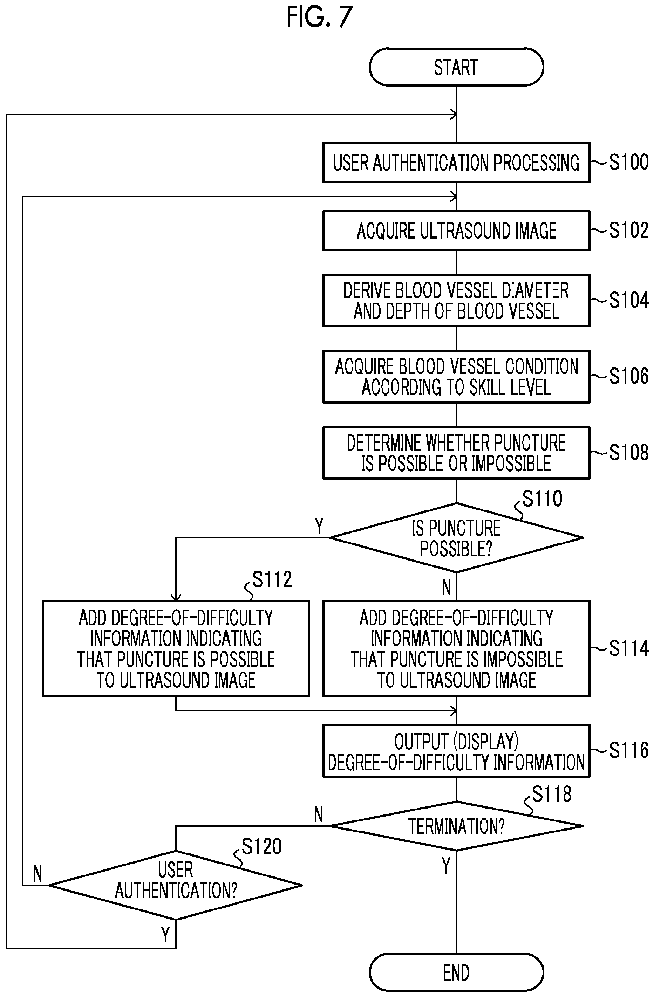

As an example, in the body part 12 according to the present embodiment, the CPU 50A of the control unit 50 executes the puncture assist processing program 51 stored in the ROM 50B to execute puncture assist processing shown in Fig. 7. Fig. 7 shows a flowchart showing an example of a flow of the puncture assist processing executed in the body part 12 according to the present embodiment. The puncture assist processing shown in Fig. 7 is executed, for example, in a case in which a power of the body part 12 is turned on or a case in which the execution is instructed by the user through the input I/F unit 56.

In step S100 of Fig. 7, the authentication unit 40 performs user authentication processing. In the present embodiment, the authentication unit 40 performs the user authentication, acquires the user ID, and outputs the acquired user ID to the determination unit 38, as described above.

In next step S102, the acquisition unit 34 acquires the ultrasound image U generated by the image generation unit 32, as described above. The acquisition unit 34 outputs the acquired ultrasound image U to the detection unit 36 and the degree-of-difficulty information output unit 44.

In next step S104, the detection unit 36 derives the blood vessel diameter and the depth of the blood vessel B in the ultrasound image U. In the present embodiment, the detection unit 36 detects the blood vessel B in the ultrasound image U and derives the blood vessel diameter and the depth of the detected blood vessel B, as described above. It should be noted that, in a case in which the ultrasound image U includes the plurality of blood vessels B as described above, the detection unit 36 derives the blood vessel diameter and the depth for each of the plurality of blood vessels B. The blood vessel diameter and the depth derived by the detection unit 36 are output to the determination unit 38.

In next step S106, the determination unit 38 acquires the blood vessel condition according to the skill level of the user. It should be noted that, in the present embodiment, as described above, the determination unit 38 acquires the minimum blood vessel diameter and the maximum blood vessel depth, which correspond to the user ID authenticated in step S100 with reference to the personal skill level information 42A and the blood vessel condition information 42B which are stored in the storage unit 52. Specifically, the determination unit 38 specifies the skill level corresponding to the user ID acquired in step S100 with reference to the personal skill level information 42A, as described above. Further, the determination unit 38 derives the minimum blood vessel diameter and the maximum blood vessel depth, which correspond to the skill level of the user, with reference to the blood vessel condition information 42B.

In next step S108, the determination unit 38 determines whether the puncture is possible or impossible. In the present embodiment, as described above, it is determined whether the puncture of the blood vessel B is possible or impossible by comparing the blood vessel diameter and the depth which are derived in step S104 for the blood vessel B in the ultrasound image U detected in step S104 with the minimum blood vessel diameter and the maximum blood vessel depth which are the blood vessel conditions according to the skill level of the user acquired in step S106.

For example, a case will be described in which the blood vessel diameter of the blood vessel B derived in step S104 is 3 mm and the depth is 9 mm. In a case in which the user ID acquired by the user authentication processing in step S100 is "0001", it is specified that the skill level is "4" in step S106 with reference to the personal skill level information 42A (see Fig. 5A). Also, "2 mm" is derived as the minimum blood vessel diameter and "20 mm" is derived as the maximum blood vessel depth with reference to the blood vessel condition information 42B (see Fig. 5B). The blood vessel diameter of the blood vessel B is 3 mm, and is larger than the minimum blood vessel diameter. Also, the depth of the blood vessel B is 9 mm, and is smaller than the maximum blood vessel depth. Therefore, the determination unit 38 determines that the puncture is possible. On the other hand, in a case in which the user ID acquired by the user authentication processing in step S100 is "0002", it is specified that the skill level is "1" in step S106 with reference to the personal skill level information 42A (see Fig. 5A). Also, "5 mm" is derived as the minimum blood vessel diameter and "10 mm" is derived as the maximum blood vessel depth with reference to the blood vessel condition information 42B (see Fig. 5B). The blood vessel diameter of the blood vessel B is 3 mm, and is smaller than the minimum blood vessel diameter. Also, the depth of the blood vessel B is 9 mm, and is smaller than the maximum blood vessel depth. Therefore, the determination unit 38 determines that the puncture is impossible. The determination result of the determination unit 38 is output to the degree-of-difficulty information output unit 44.

In next step S110, the degree-of-difficulty information output unit 44 determines whether the puncture is possible or impossible for the determination result of the determination unit 38. In a case of the determination result that the puncture is possible, an affirmative determination is made in the determination in step S110, and the processing proceeds to step S112.

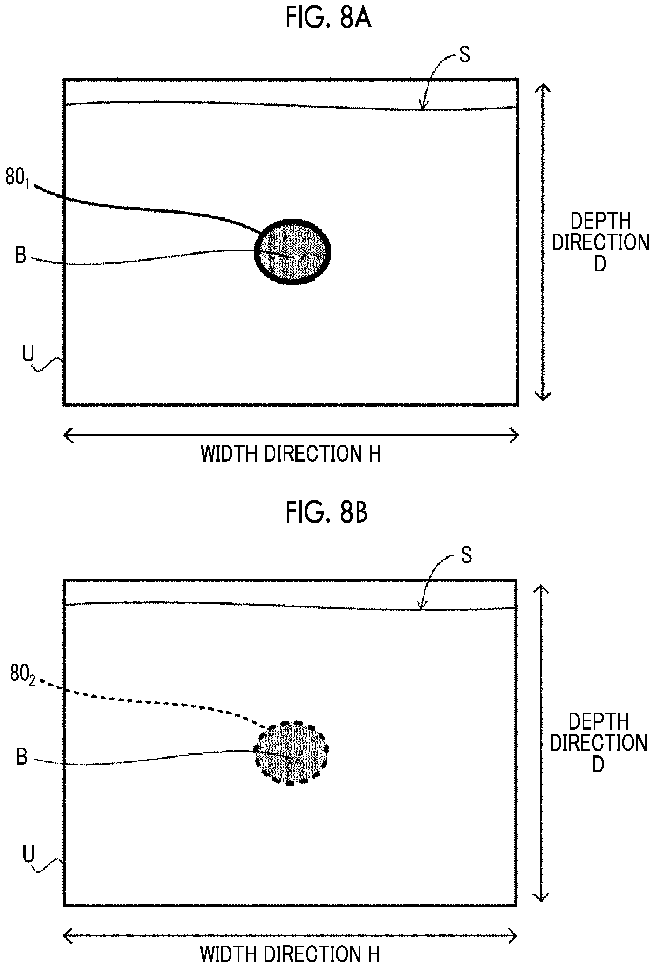

In step S112, as described above, after the degree-of-difficulty information output unit 44 adds the degree-of-difficulty information indicating that the puncture is possible to the ultrasound image U acquired in step S102, the processing proceeds to step S116. Fig. 8A shows an example of the ultrasound image U to which the degree-of-difficulty information indicating that the puncture is possible is added. In the example shown in Fig. 8A, a form example is shown in which a relatively thick solid line enclosing an outer shape of the blood vessel B is applied as degree-of-difficulty information 80₁ indicating that the puncture is possible.

On the other hand, in a case of the determination result that the puncture is impossible, a negative determination is made in the determination in step S110, and the processing proceeds to step S114. In step S114, as described above, after the degree-of-difficulty information output unit 44 adds the degree-of-difficulty information indicating that the puncture is impossible to the ultrasound image U acquired in step S102, the processing proceeds to step S116. Fig. 8B shows an example of the ultrasound image U to which the degree-of-difficulty information indicating that the puncture is impossible is added. In the example shown in Fig. 8B, a form example is shown in which a relatively thick dotted line enclosing the outer shape of the blood vessel B is applied as degree-of-difficulty information 80₂ indicating that the puncture is impossible.

In next step S116, the degree-of-difficulty information output unit 44 outputs the ultrasound image U to which the degree-of-difficulty information generated in step S112 or step S114 is added, to the display unit 46. As a result, as in Figs. 8A and 8B, the ultrasound image U to which the degree-of-difficulty information 80₁ or 80₂ is added is displayed on the display unit 46. It should be noted that, in the present embodiment, the form has been shown in which the types of the line (solid line or dotted line) as the display forms are made different for the degree-of-difficulty information 80₁ in a case in which the puncture is possible and the degree-of-difficulty information 80₂ in a case in which the puncture is impossible, but a form may be adopted in which other display forms are made different without being limited to the type of the line. For example, a form may be adopted in which display forms, such as hue of the line, chroma saturation of the line, shading of the line, thickness of the line, and the presence or absence of turning on and off, are made different. In addition, a form may be adopted in which a frame enclosing a region of the blood vessel B is provided so that the display forms, such as a shape or a hue, are made different for the frame. In addition, a form may be adopted in which an arrow or a mark indicating whether the puncture is possible or impossible is displayed.

In next step S118, the degree-of-difficulty information output unit 44 determines whether or not to terminate the puncture assist processing. In the present embodiment, the puncture assist processing shown in Fig. 7 is terminated in a case in which a predetermined termination condition is satisfied, such as a case in which the user instructs the termination through the input I/F unit 56, a case in which the power of the body part 12 is cut off, or a case in which a predetermined time has elapsed after the processing of step S116 is terminated. In a case in which the termination condition is not satisfied, a negative determination is made in the determination in step S118, and the processing proceeds to step S120.

In a case in which the degree-of-difficulty information added to the ultrasound image U displayed on the display unit 46 by the processing of step S116 corresponds to the case in which the puncture is impossible, for example, the user who performs the puncture may be changed from the user currently authenticated to another user, for example, the user having a high skill level. In such a case, in order to perform the user authentication again for the changed user, the authentication unit 40 determines whether or not to perform the user authentication in step S120. In a case in which the user authentication is performed, an affirmative determination is made in the determination in step S120, the processing returns to step S100, and the processing of steps S100 to S118 is repeated.

On the other hand, in a case in which the degree-of-difficulty information added to the ultrasound image U displayed on the display unit 46 by the processing of step S116 corresponds to the case in which the puncture is impossible, for example, the puncture position may be changed. In such a case, it is determined whether the puncture of the blood vessel B in the ultrasound image U captured at the changed position is possible or impossible. Therefore, a negative determination is made in the determination in step S120, and the processing proceeds to step S102, and the processing of steps S102 to S118 is repeated.

On the other hand, in a case in which the termination condition is satisfied in step S118, the puncture assist processing shown in Fig. 7 is terminated.

It should be noted that the present invention is not limited to the present form, and the following modification examples can be applied, for example.

### [Modification Example 1]

In the form described above, the form has been described in which the minimum blood vessel diameter and the maximum blood vessel depth are applied as the blood vessel conditions, but in the present modification example, other blood vessel conditions will be described.

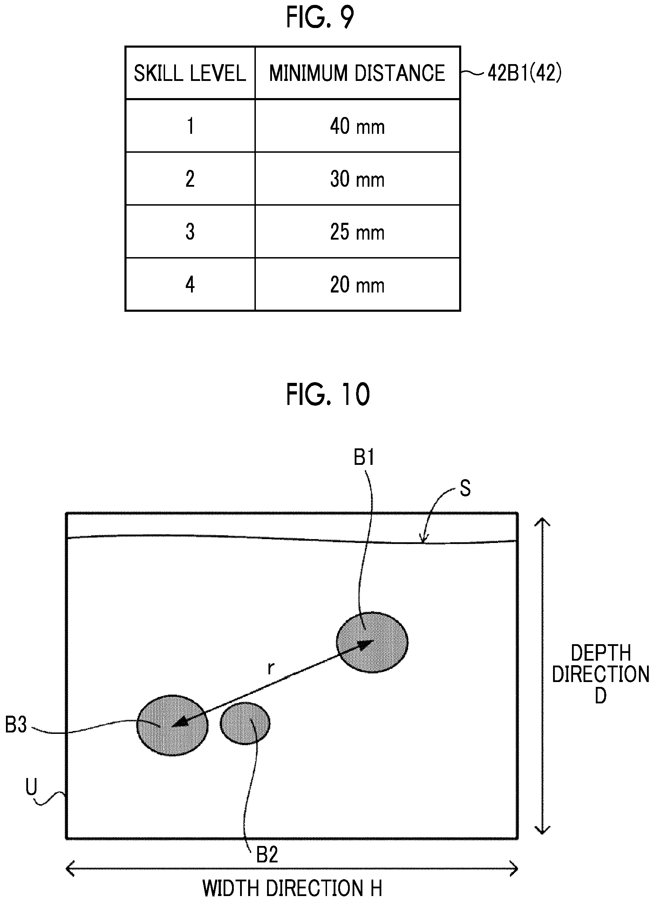

One factor that determines the degree of difficulty of the puncture of the blood vessel B is a distance between the blood vessel B that is a target for the puncture, and another tissue that is not the target for the puncture. In general, as the distance between the blood vessel B and the other tissue is smaller, it is more difficult to perform the puncture, and the difficulty of the puncture is higher. On the other hand, as the distance between the blood vessel B and the other tissue is larger, it is easier to perform the puncture, and the degree of difficulty of the puncture is lower, in other words, a degree of easiness of the puncture is higher. Therefore, in the present modification example, the minimum distance between the blood vessel B and the other tissue at which puncture is possible is determined as the blood vessel condition for each skill level. Fig. 9 shows an example of blood vessel condition information 42B1 according to the present modification example. In the present modification example, the skill level information 42 stored in the storage unit 52 includes the blood vessel condition information 42B1 shown in Fig. 9 instead of the blood vessel condition information 42B (see Fig. 5B) according to the form described above. The minimum distance in the present modification example is an example of a spacing threshold value according to the present disclosure. It should be noted that the other tissue that is not the target for the puncture, includes organs, such as nerves, tendons, and muscles surrounding the blood vessel B that is the target for the puncture, as well as blood vessels such as surrounding arteries and veins. In addition, in the following description, the blood vessel B that is the target for the puncture will be referred to as a "target blood vessel", and the blood vessel B that is not the target for the puncture will be referred to as a "non-target blood vessel".

For example, as shown in Fig. 10, a case will be described in which a blood vessel B1 that is a basilic vein, a blood vessel B2 that is a brachial vein, and a blood vessel B3 that is a brachial artery are present, and the blood vessel B1 is the target blood vessel. In this case, the blood vessel B3 is the non-target blood vessel. In this case, the determination unit 38 determines that the puncture is possible in a case in which a distance r between the blood vessel B1 and the blood vessel B3 is equal to or larger than the minimum distance. In other words, the determination unit 38 determines that the puncture is impossible in a case in which the distance r between the blood vessel B1 and the blood vessel B3 is smaller than the minimum distance. Specifically, in a case in which the skill level of the user is "2", the determination unit 38 determines that the puncture is possible in a case in which the distance r between the blood vessel B1 and the blood vessel B3 is equal to or larger than 30 mm, and determines that the puncture is impossible in a case in which the distance r is smaller than 30 mm. In addition, for example, in a case in which the distance r between the blood vessel B1 and the blood vessel B3 is 30 mm, the determination unit 38 determines that the puncture is impossible in a case in which the skill level is "1", and determines that the puncture is possible in a case in which the skill level is equal to or higher than "2".

As described above, in the present modification example, since the distance r between the blood vessel B that is the target for the puncture and the other tissue that is not the target for the puncture is determined as the blood vessel condition, the detection unit 36 detects the tissue surrounding the blood vessel B that is the target blood vessel from the ultrasound image U, and derives the distance r between the blood vessel B that is the target blood vessel and the other tissue. In a case of the example shown in Fig. 10, the distance r between the blood vessel B1 and the blood vessel B3 is detected. It should be noted that the method in which the detection unit 36 detects the tissue surrounding the target blood vessel from the ultrasound image U is not particularly limited. As an example, the detection unit 36 according to the present modification example detects the tissue surrounding the target blood vessel in the ultrasound image U by analyzing the ultrasound image U acquired by the acquisition unit 34 according to a known algorithm. For example, the detection unit 36 can store the typical pattern data of the blood vessel region including the tissue surrounding the target blood vessel, in the example shown in Fig. 10, the blood vessel B3 that is the brachial artery, as the template in advance, derive a degree of similarity with respect to the pattern data while searching the ultrasound image U with the template, and consider that the blood vessel B3 is present in a place in which the degree of similarity is equal to or larger than the reference value and is maximized.

Also, in addition to simple template matching, the derivation of the degree of similarity includes a method using a trained learning model based on a feature amount of an image showing the surrounding tissue. For example, a machine learning method such as SVM or AdaBoost described above, or a general image recognition method using deep learning described above can be used.

In addition, for example, the detection unit 36 may detect the tissue surrounding the target blood vessel in the ultrasound image U using a tissue detection model which is a trained model that has been subjected to machine learning using a plurality of ultrasound images U labeled with respect to the surrounding tissue. For example, the tissue detection model is an object detection algorithm using deep learning. As the tissue detection model, for example, an object detection model configured by a regional convolutional neural network (R-CNN), which is a type of a CNN, can be used. The tissue detection model detects the tissue as an object from the input ultrasound image U, and outputs information indicating the tissue surrounding the target blood vessel in the ultrasound image U.

Further, as described above, the detection unit 36 according to the present modification example has a function of deriving the distance r between the blood vessel B that is the target blood vessel and the detected surrounding tissue. It should be noted that the method in which the detection unit 36 derives the distance r between the blood vessel B, which is the target blood vessel in the ultrasound image U, and the surrounding tissue is not particularly limited. As an example, the detection unit 36 according to the modification example applies known image analysis processing to the blood vessel B in the detected ultrasound image U to derive the distance r. In the example shown in Fig. 9, the detection unit 36 derives the distance r between the center of the blood vessel B1 that is the target blood vessel and the center of the blood vessel B3 of the surrounding tissue that is the non-target blood vessel by the image analysis processing.

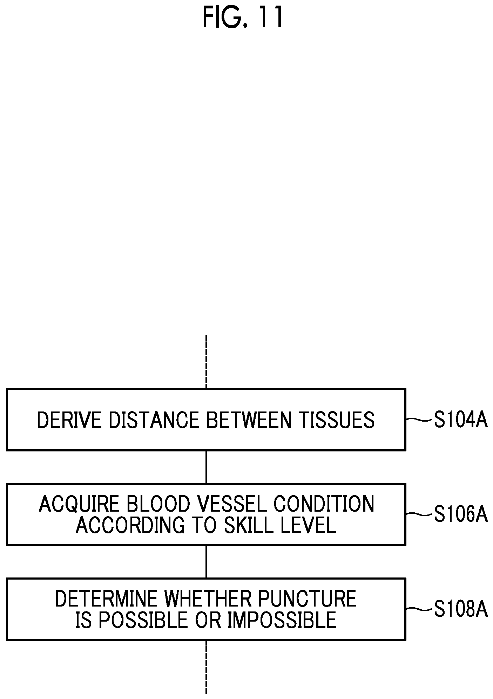

Fig. 11 shows a flowchart showing an example of a flow of the puncture assist processing according to the present modification example. It should be noted that, since the processing of steps S100 to S102 and the processing after steps S110 are the same as the puncture assist processing according to the form described above (see Fig. 7), the description thereof is omitted.

As shown in Fig. 11, in the present modification example, in step S104A, the detection unit 36 derives the distance r between the tissue of the blood vessel B that is the target blood vessel in the ultrasound image U, and the surrounding tissue, as described above. As described above, the detection unit 36 detects the blood vessel B that is the target blood vessel in the ultrasound image U and the surrounding tissue, and derives the distance r between the blood vessel B and the surrounding tissue, which are detected. It should be noted that, in a case in which the ultrasound image U includes the plurality of blood vessels B that are the target blood vessels as described above, the detection unit 36 derives the distance r for each of the plurality of blood vessels B. The distance r derived by the detection unit 36 is output to the determination unit 38.

In next step S106A, the determination unit 38 acquires the blood vessel condition according to the skill level of the user. It should be noted that, in the present modification example, as described above, the determination unit 38 acquires the minimum distance corresponding to the user ID authenticated in step S100 with reference to the personal skill level information 42A and the blood vessel condition information 42B1 which are stored in the storage unit 52. Specifically, the determination unit 38 specifies the skill level corresponding to the user ID acquired in step S100 with reference to the personal skill level information 42A, as described above. Further, the determination unit 38 derives the minimum distance corresponding to the skill level of the user, with reference to the blood vessel condition information 42B1.

In next step S108A, the determination unit 38 determines whether the puncture is possible or impossible, as described above. In the present modification example, as described above, it is determined whether the puncture of the blood vessel B is possible or impossible by comparing the distance r derived in step S104A for the blood vessel B that is the target blood vessel in the ultrasound image U detected in step S104A with the minimum distance which is the blood vessel condition according to the skill level of the user acquired in step S106A.

As described above, in the present modification example, the form has been described in which the minimum distance between the target blood vessel and the surrounding tissue is applied as the blood vessel condition, but the blood vessel condition is not limited to the present modification example. In addition, each of the minimum blood vessel diameter and the maximum blood vessel depth which are the blood vessel conditions in the form described above, and the minimum distance according to the present modification example may be used alone as the blood vessel condition, or may be used in combination as the blood vessel conditions.

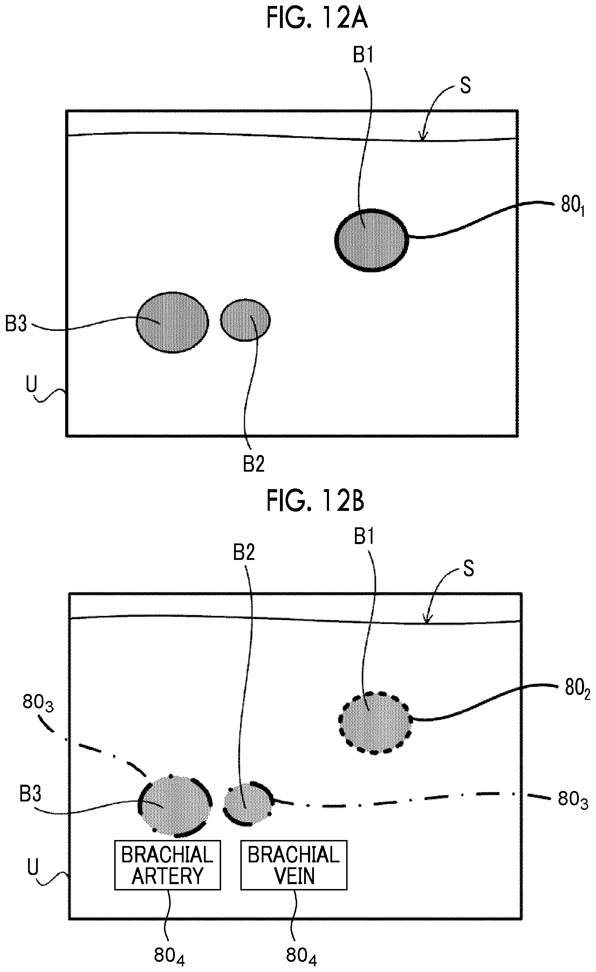

Fig. 12A shows an example of the ultrasound image U to which the degree-of-difficulty information 80₁ indicating that the puncture is possible in the present modification example is added. In the example shown in Fig. 12A, a display example of the ultrasound image U in a case in which the blood vessel B1 is the target blood vessel and the puncture of the blood vessel B1 is possible is shown. As described above, even in the present modification example, since the degree-of-difficulty information 80₁ is added to the blood vessel B1 in which the puncture is possible, the user can perform the puncture with reference to the degree-of-difficulty information 80₁.

On the other hand, Fig. 12B shows an example of the ultrasound image U to which the degree-of-difficulty information 80₂ indicating that the puncture is impossible in the present modification example is added. In the example shown in Fig. 12B, a display example in a case in which the blood vessel B1 is the target blood vessel and the puncture of the blood vessel B1 is impossible is shown. In addition, in the present modification example, as shown in Fig. 12B, in a case in which the puncture of the target blood vessel is impossible, the degree-of-difficulty information output unit 44 information 80₃ indicating the surrounding tissue which is the reason why the puncture is impossible, as well as information 80₁ indicating the name of the tissue are added to the ultrasound image U and displayed on the display unit 46. It should be noted that, in the example shown in Fig. 12B, a form example is shown in which relatively thick one-dot chain lines enclosing the outer shapes of the blood vessels B2 and B3 are applied as the information 80₃ indicating the surrounding tissue. As described above, even in the present modification example, since the degree-of-difficulty information 80₂ is added to the blood vessel B1 in which the puncture is impossible, the user can recognize that the puncture is impossible with reference to the degree-of-difficulty information 80₂. In addition, in the present modification example, since the information 80₃ and 80₄ are also added to the surrounding tissue, the user can recognize the reason why the puncture is impossible. It should be noted that a form may be adopted in which any one of the information 80₃ or 80₄ is added to the ultrasound image U and displayed. In addition, even in a case in which the puncture is possible, a form may be adopted in which the information 80₃ and 80₄ are displayed. In addition, a form may be adopted in which the information 80₃ and 80₄ are displayed according to the skill level of the user. For example, a form may be adopted in which the information 80₃ and 80₄ are displayed in a case in which the skill level of the user is relatively low, and it is determined whether or not to display the information 80₃ and 80₄ by the selection of the user in a case in which the skill level of the user is relatively high.

### [Modification Example 2]

In the form described above, the form has been described in which the skill level of the user is set by the administrator or the like, but another example of the method of setting the skill level will be described in the present modification example. As an example, in the present modification example, a form will be described in which a preset skill level is corrected based on a puncture record.

For example, it can be said that the skill level of the user is higher as the number of punctures is larger. Therefore, the determination unit 38 in a case in which the number of punctures is considered as the skill level accumulates the number of punctures in association with the user ID. In addition, in a case in which it is determined whether the puncture is possible or impossible in step S108 of the puncture assist processing (see Fig. 7), a form may be adopted in which the determination unit 38 performs correction to increase the skill level of the user acquired with reference to the personal skill level information 42A to a predetermined degree in a case in which the number of punctures exceeds a threshold value, and acquires the blood vessel condition with reference to the blood vessel condition information 42B based on the corrected skill level.

In addition, for example, it can be said that the skill level of the user is higher as the number of times that the puncture is successful is larger. On the contrary, it can be said that the skill level of the user is lower as the number of times that the puncture fails is larger. It should be noted that, in this case, the "success" and the "failure" of the puncture are determined by whether or not the puncture needle N is appropriately inserted into the blood vessel B that is the target blood vessel, the presence or absence of damage to other tissues, the presence or absence of the blood outflow to the outside of the blood vessel B, whether or not the puncture needle N penetrates the blood vessel B, and the like. Therefore, in a case in which the success or the failure of the puncture is considered as the skill level, the determination unit 38 determines whether the user succeeds or fails in the puncture and accumulates the determination result. It should be noted that the method in which the determination unit 38 determines the success or the failure of the puncture is not particularly limited. For example, a form may be adopted in which the determination unit 38 detects the puncture needle N from the ultrasound image U and determine the success or the failure of the puncture by identifying a relationship between the detected puncture needle N and the blood vessel B. It should be noted that the method in which the determination unit 38 detects the puncture needle N from the ultrasound image U is not particularly limited, and for example, the same method as the method in which the blood vessel B is detected from the ultrasound image U can be applied. For example, it is possible to store typical pattern data of the puncture needle N as a template in advance, derive a degree of similarity with respect to the pattern data while searching the ultrasound image U with the template, and consider that the puncture needle N is present in a place in which the degree of similarity is equal to or larger than a reference value and is maximized. In addition, in a case in which it is determined whether the puncture is possible or impossible in step S108 of the puncture assist processing (see Fig. 7), the determination unit 38 in this case performs correction to increase the skill level of the user acquired with reference to the personal skill level information 42A to a predetermined degree in a case in which the number of times that the puncture is successful exceeds the threshold value, and acquires the blood vessel condition with reference to the blood vessel condition information 42B based on the corrected skill level. On the other hand, a form may be adopted in which, in a case in which the number of times that the puncture fails exceeds the threshold value, the correction to decrease the skill level of the user acquired with reference to the personal skill level information 42A to a predetermined degree is performed, and the blood vessel condition is acquired with reference to the blood vessel condition information 42B based on the corrected skill level.

In addition, for example, it is estimated that the skill level of the user is higher as a time required for the puncture is shorter. On the contrary, it is estimated that the skill level of the user is lower as the time required for the puncture is longer. It should be noted that the time required for the puncture may be, for example, an elapsed time from the start of scanning the subject with the ultrasound probe 10 to the termination of the puncture. In addition, for example, an elapsed time from the insertion of the puncture needle N into the subject to the removal of the puncture needle N to the outside from the subject may be used. Therefore, in a case in which the time required for the puncture is considered as the time required for the skill level, the determination unit 38 counts the time required for the puncture and accumulates information (for example, the number of times that the time required for the puncture is shorter than the threshold value time) indicating whether or not the time required for the puncture is shorter than the threshold value time. In a case in which it is determined whether the puncture is possible or impossible in step S108 of the puncture assist processing (see Fig. 7), the determination unit 38 in this case may perform the correction to increase the skill level of the user acquired with reference to the personal skill level information 42A to a predetermined degree in a case in which the number of times that the time required for the puncture is shorter than the threshold value time exceeds a threshold value number of times, and acquire the blood vessel condition with reference to the blood vessel condition information 42B based on the corrected skill level.

### [Modification Example 3]

In the present modification example, a form will be described in which the determination unit 38 determines whether the puncture is possible or impossible in further consideration of an image quality of the ultrasound image U.

In a case in which the skill level of the user is high, since the user is familiar with capturing the ultrasound image U by the ultrasound probe 10, it can be said that the image quality of the captured ultrasound image U, specifically, the ultrasound image U generated by the image generation unit 32 is relatively high. In this case, the determination unit 38 considers that the image quality is high in a case in which the contrast, the gain, and the presence or absence of the noise as the image quality of the ultrasound image U generated by the image generation unit 32 are equal to or higher than the predetermined reference, and considers that the skill level of the user who captures the ultrasound image U is high. In a case in which it is determined whether the puncture is possible or impossible in step S108 of the puncture assist processing (see Fig. 7), the determination unit 38 in this case may determine the image quality of the ultrasound image U, perform the correction to increase the skill level of the user acquired with reference to the personal skill level information 42A to a predetermined degree in a case in which the image quality is equal to or higher than a predetermined reference, and acquire the blood vessel condition with reference to the blood vessel condition information 42B based on the corrected skill level. On the other hand, in a case in which the determined image quality of the ultrasound image U is lower than the predetermined reference, the determination unit 38 may perform the correction to decrease the skill level of the user acquired with reference to the personal skill level information 42A to a predetermined degree, and acquire the blood vessel condition with reference to the blood vessel condition information 42B based on the corrected skill level. It should be noted that a form may be adopted in which the skill level is determined from the image quality of the ultrasound image U instead of performing the user authentication and acquiring the skill level associated with the user ID.

In addition, in a case in which the image quality of the captured ultrasound image U, specifically, the ultrasound image U generated by the image generation unit 32 is relatively high, the ultrasound image U displayed on the display unit 46 is relatively easy-to-see image, and thus there is a tendency that the puncture is easy. Therefore, in a case in which the image quality of the ultrasound image U is equal to or higher than the predetermined reference, even the user having a lower skill level than the user according to the form described above may be considered to be appropriate for the puncture. In this case, the determination unit 38 considers that the image quality is high in a case in which the contrast, the gain, and the presence or absence of the noise as the image quality of the ultrasound image U generated by the image generation unit 32 are equal to or higher than the predetermined reference, and includes the skill level of the user who performs the puncture with reference to the ultrasound image U within a range lower than the original skill level. In a case in which it is determined whether the puncture is possible or impossible in step S108 of the puncture assist processing (see Fig. 7), the determination unit 38 in this case may determine the image quality of the ultrasound image U, perform the correction to decrease the skill level of the user acquired with reference to the personal skill level information 42A to a predetermined degree in a case in which the image quality is equal to or higher than a predetermined reference, and acquire the blood vessel condition with reference to the blood vessel condition information 42B based on the corrected skill level. It should be noted that a form may be adopted in which the blood vessel condition shown in the blood vessel condition information 42B is relaxed instead of decreasing the skill level.

As described above, the body part 12 according to each form described above comprises the acquisition unit 34 that acquires the ultrasound image U of the tissue including the blood vessel B of the subject, the detection unit 36 that detects the blood vessel B from the acquired ultrasound image U, and the degree-of-difficulty information output unit 44 that outputs the degree-of-difficulty information related to the degree of difficulty of the puncture in the detected blood vessel B according to the skill level of the user who performs the puncture.

Therefore, with the body part 12 according to each form described above, it is possible to present the information indicating an appropriate degree of difficulty of the puncture according to the user who performs the puncture of the blood vessel, as described above. In addition, with the body part 12 according to each form described above, since the user who performs the puncture can intuitively recognize the degree of difficulty of the puncture, it is easy to specify an appropriate blood vessel B for the puncture or the position of the blood vessel.

It should be noted that the technology according to the present disclosure is not limited to each form described above, and further various modifications can be made.

For example, a form may be adopted in which the skill level of the user can be changed or set according to a user's condition of the day. In addition, a form may be adopted in which the blood vessel condition is displayed on the display unit 46 according to the skill level of the user, and the displayed blood vessel condition can be adjusted by operating the input I/F unit 56 by the user or the like.

In addition, a form may be adopted in which information indicating a warning is output in a case in which the user attempts to perform the puncture of the blood vessel B for which it is determined that the puncture is impossible. In this case, the determination unit 38 detects the puncture needle N in the ultrasound image U in the echo-guided puncture, determines whether or not the blood vessel B for which it is determined that the puncture is impossible is present on the extension line of the detected puncture needle N, and outputs, in a case in which the blood vessel B is present, the presence as the determination result to the degree-of-difficulty information output unit 44. In addition, a form may be adopted in which the degree-of-difficulty information output unit 44 displays the information indicating the warning on the display unit 46 in a case in which the determination result of the determination unit 38 is input. It should be noted that the warning display in this case may be any a visible display or an audible display.

In addition, in the form described above, the form has been described in which the body part 12 is an example of the information processing apparatus according to the present disclosure, but a device other than the body part 12 may have the function of the information processing apparatus according to the present disclosure. In other words, a device other than the body part 12, for example, the ultrasound probe 10 or an external device may have a part or all of the functions of the acquisition unit 34, the detection unit 36, the determination unit 38, the authentication unit 40, and the degree-of-difficulty information output unit 44.

In addition, in each form described above, the body part 12 is provided with the image generation unit 32 that generates the ultrasound image U based on the sound ray signal, but the image generation unit 32 may be provided in the ultrasound probe 10 instead of the configuration described above. In this case, the ultrasound probe 10 generates the ultrasound image U and outputs the ultrasound image U to the body part 12. The CPU 50A of the control unit 50 of the body part 12 performs the puncture assist processing or the like based on the ultrasound image U input from the ultrasound probe 10.

In addition, in each form described above, the form has been described in which the body part 12 comprises the display unit 46, the input I/F unit 56, and the ultrasound probe 10, but the display unit 46, the input I/F unit 56, the ultrasound probe 10, and the control unit 50 may be indirectly connected via the network.

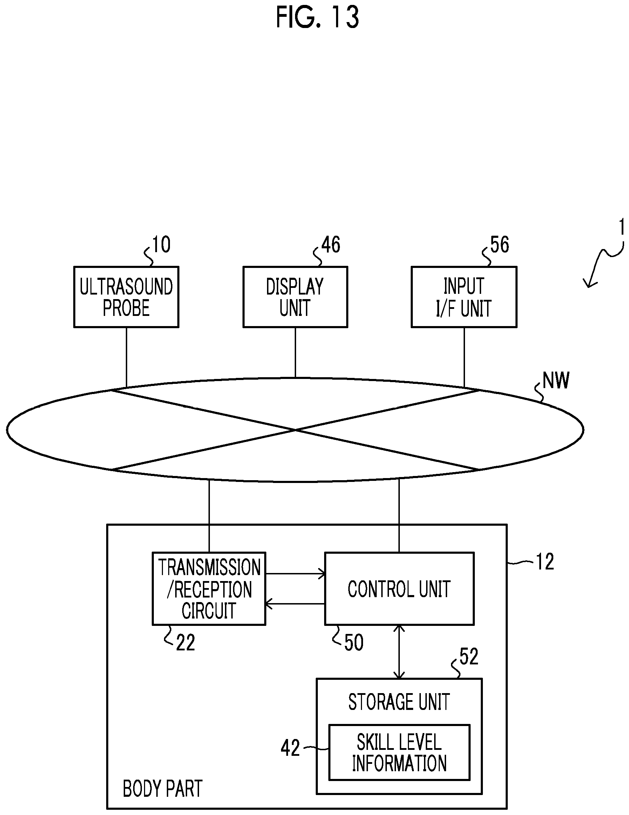

As an example, in the ultrasound diagnostic apparatus 1 shown in Fig. 13, the display unit 46, the input I/F unit 56, and the ultrasound probe 10 are connected to the body part 12 through a network NW. The body part 12 is obtained by removing the display unit 46 and the input I/F unit 56 from the body part 12 according to the form described above shown in Fig. 1 and adding the transmission/reception circuit 22, and comprises the transmission/reception circuit 22, the control unit 50, and the storage unit 52. The ultrasound probe 10 is obtained by removing the transmission/reception circuit 22 from the ultrasound probe 10 according to the form described above shown in Fig. 1.

As described above, in the ultrasound diagnostic apparatus 1 shown in Fig. 13, since the display unit 46, the input I/F unit 56, and the ultrasound probe 10 are connected to the body part 12 through the network NW, the body part 12 can be used as a so-called remote server. As a result, for example, the user can prepare the display unit 46, the input I/F unit 56, and the ultrasound probe 10 at the user's hand, and thus the convenience is improved. In addition, by configuring the display unit 46 and the input I/F unit 56 with the portable terminal, such as the smartphone or the tablet terminal, the convenience is further improved.

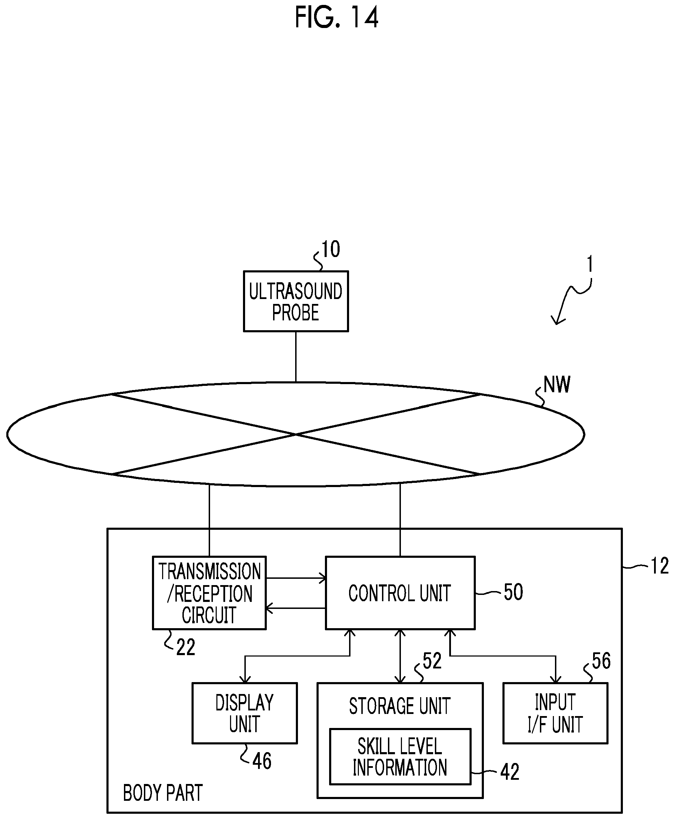

As another example, in the ultrasound diagnostic apparatus 1 shown in Fig. 14, the body part 12 comprises the display unit 46 and the input I/F unit 56, and the ultrasound probe 10 is connected to the body part 12 through the network NW. In this case, the body part 12 may be configured by the remote server. In addition, the body part 12 can also be configured by the portable terminal, such as the smartphone or the tablet terminal.

In addition, in the form described above, various processors shown below can be used as the hardware structure of processing units that execute various types of processing, such as the acquisition unit 34, the detection unit 36, the determination unit 38, the authentication unit 40, and the degree-of-difficulty information output unit 44. As described above, the various processors include, in addition to the CPU that is a general-purpose processor which executes software (program) and functions as various processing units, a programmable logic device (PLD) that is a processor of which a circuit configuration can be changed after manufacture, such as a field programmable gate array (FPGA), and a dedicated electric circuit that is a processor having a circuit configuration which is designed for exclusive use in order to execute specific processing, such as an application specific integrated circuit (ASIC).

One processing unit may be configured by one of the various processors, or may be configured by a combination of two or more processors of the same type or different types (for example, a combination of a plurality of FPGAs or a combination of the CPU and the FPGA). Also, a plurality of processing units may be configured by one processor.

A first example of the configuration in which the plurality of processing units are configured by one processor is a form in which, as represented by computers, such as a client and a server, one processor is configured by a combination of one or more CPUs and the software and this processor functions as the plurality of processing units. Second, there is a form in which, as represented by a system on chip (SoC) or the like, a processor that realizes the function of the entire system including the plurality of processing units by one integrated circuit (IC) chip is used. As described above, as the hardware structure, the various processing units are configured by using one or more of the various processors described above.

Further, more specifically, an electric circuit (circuitry) in which circuit elements, such as semiconductor elements, are combined can be used as the hardware structure of the various processors.

In addition, in the embodiment described above, the aspect has been described in which the puncture assist processing program 51 is stored (installed) in the ROM 50B in advance, but the present invention is not limited to this. Each of the puncture assist processing program 51 may be provided in a form of being recorded in a recording medium, such as a compact disc read only memory (CD-ROM), a digital versatile disc read only memory (DVD-ROM), and a universal serial bus (USB) memory. In addition, a form may be adopted in which each of the puncture assist processing program 51 is provided in a form being downloaded from an external device through a network.

### Explanation of References

1: ultrasound diagnostic apparatus
10: ultrasound probe
12: body part
20: oscillator array
22: transmission/reception circuit
24: transmission circuit
26: reception circuit
30: imaging control unit
32: image generation unit
34: acquisition unit
36: detection unit
38: determination unit
40: authentication unit
42: skill level information, 42A: personal skill level information, 42B, 42B1: blood vessel condition information
44: degree-of-difficulty information output unit
46: display unit
50: control unit, 50A: CPU, 50B: ROM, 50C: RAM
51: puncture assist processing program
52: storage unit
54: communication I/F unit
56: input I/F unit
59: bus
60: amplification unit
62: A/D conversion unit
64: beam former
70: signal processing unit
72: DSC
74: image processing unit
80₁, 80₂: degree-of-difficulty information
B, B1 to B3: blood vessel
D: depth direction
d1, d2: length
H: width direction
N: puncture needle
NW: network
r: distance
S: body surface
U: ultrasound image

## Claims

1. An information processing apparatus comprising:
an acquisition unit that acquires an ultrasound image of a tissue including a blood vessel of a subject;
a detection unit that detects the blood vessel from the acquired ultrasound image; and
**characterized in that** it further comprises:
a degree-of-difficulty information output unit that outputs degree-of-difficulty information related to a degree of difficulty of a puncture in the detected blood vessel according to a skill level of a user who performs the puncture.

2. The information processing apparatus according to claim 1, further comprising:
a determination unit that determines whether the puncture is possible or impossible based on a detection result of the detection unit,
wherein the degree-of-difficulty information output unit uses a determination result of the determination unit as the degree-of-difficulty information.

3. The information processing apparatus according to claim 2, wherein the determination unit determines whether the puncture is possible or impossible based on a blood vessel condition related to whether the puncture is possible or impossible according to the skill level of the user, and the detection result.

4. The information processing apparatus according to claim 2 or 3, wherein, in a case in which an image quality of the ultrasound image is higher than a predetermined image quality, the determination unit relaxes the blood vessel condition in which the puncture is possible.

5. The information processing apparatus according to any one of claims 2 to 4, wherein:
the detection unit further detects a depth from a body surface of the subject to the blood vessel, and
the determination unit determines whether the puncture is possible or impossible based on the depth.

6. The information processing apparatus according to claim 5, wherein, in a case in which the depth is smaller than a depth threshold value, the determination unit determines that the puncture is possible.

7. The information processing apparatus according to any one of claims 2 to 6, wherein:
the detection unit further detects a blood vessel diameter indicating a thickness of the blood vessel, and
the determination unit determines whether the puncture is possible or impossible based on the blood vessel diameter.

8. The information processing apparatus according to claim 7, wherein, in a case in which the blood vessel diameter is larger than a blood vessel diameter threshold value, the determination unit determines that the puncture is possible.

9. The information processing apparatus according to any one of claims 2 to 8, wherein
the detection unit further detects a tissue surrounding the blood vessel, and
the determination unit determines whether the puncture is possible or impossible based on the tissue.

10. The information processing apparatus according to claim 9, wherein, in a case in which a distance between the blood vessel and the tissue is equal to or larger than a spacing threshold value, the determination unit determines that the puncture is possible.

11. The information processing apparatus according to claim 9 or 10, wherein the blood vessel is a target blood vessel that is a target for the puncture, and the tissue includes a non-target blood vessel that is not the target for the puncture.

12. The information processing apparatus according to any one of claims 2 to 11, wherein the degree-of-difficulty information is information of which a display form differs depending on whether the puncture is possible or impossible.

13. The information processing apparatus according to any one of claims 2 to 12, wherein, in a case in which an image quality of the ultrasound image is higher than a predetermined reference, the determination unit increases the skill level, which is acquired, and determines whether the puncture is possible or impossible based on a blood vessel condition according to the increased skill level.

14. The information processing apparatus according to any one of claims 1 to 13, wherein the degree-of-difficulty information output unit outputs the ultrasound image to which the degree-of-difficulty information is added.

15. An ultrasound diagnostic apparatus comprising:
an ultrasound probe that receives an ultrasound echo from transmitted ultrasound, and outputs a reception signal based on the received ultrasound echo;
an image generation unit that generates an ultrasound image based on the reception signal input from the ultrasound probe; and
the information processing apparatus according to any one of claims 1 to 14.

16. An information processing method executed by a computer, the method comprising:
acquiring an ultrasound image of a tissue including a blood vessel of a subject;
detecting the blood vessel from the acquired ultrasound image; and
**characterized by**:
outputting degree-of-difficulty information related to a degree of difficulty of a puncture in the detected blood vessel according to a skill level of a user who performs the puncture.

17. An information processing program causing a computer to execute a process comprising:
acquiring an ultrasound image of a tissue including a blood vessel of a subject;
detecting the blood vessel from the acquired ultrasound image; and
**characterized by**:
outputting degree-of-difficulty information related to a degree of difficulty of a puncture in the detected blood vessel according to a skill level of a user who performs the puncture.

## Patentansprüche

1. Informationsverarbeitungsvorrichtung, umfassend:
eine Erfassungseinheit, die ein Ultraschallbild eines Gewebes einschließlich eines Blutgefäßes einer Untersuchungsperson erfasst;
eine Detektionseinheit, die das Blutgefäß aus dem erfassten Ultraschallbild detektiert; und
**dadurch gekennzeichnet, dass** es ferner umfasst:
eine Schwierigkeitsgradinformations-Ausgabeeinheit, die Schwierigkeitsgradinformationen, die sich auf einen Schwierigkeitsgrad einer Punktion an dem detektierten Blutgefäß beziehen, gemäß einem Fähigkeitsniveau eines Benutzers, der die Punktion durchführt, ausgibt.

2. Informationsverarbeitungsvorrichtung nach Anspruch 1, ferner umfassend:
eine Bestimmungseinheit, die auf der Grundlage eines Detektionsergebnisses der Detektionseinheit bestimmt, ob die Punktion möglich oder unmöglich ist,
wobei die Schwierigkeitsgradinformations-Ausgabeeinheit ein Bestimmungsergebnis der Bestimmungseinheit als die Schwierigkeitsgradinformationen verwendet.

3. Informationsverarbeitungsvorrichtung nach Anspruch 2, wobei die Bestimmungseinheit bestimmt, ob die Punktion auf der Grundlage einer Blutgefäßbedingung, die sich darauf bezieht, ob die Punktion gemäß dem Fähigkeitsniveau des Benutzers und dem Detektionsergebnisses möglich oder unmöglich ist, möglich oder unmöglich ist.

4. Informationsverarbeitungsvorrichtung nach Anspruch 2 oder 3, wobei in einem Fall, in dem eine Bildqualität des Ultraschallbildes höher als eine vorbestimmte Bildqualität ist, die Bestimmungseinheit die Blutgefäßbedingung, unter der die Punktion möglich ist, entspannt.

5. Informationsverarbeitungsvorrichtung nach einem der Ansprüche 2 bis 4, wobei:
die Detektionseinheit ferner eine Tiefe von einer Körperoberfläche der Untersuchungsperson bis zu dem Blutgefäß detektiert, und
die Bestimmungseinheit auf der Grundlage der Tiefe bestimmt, ob die Punktion möglich oder unmöglich ist.

6. Informationsverarbeitungsvorrichtung nach Anspruch 5, wobei in einem Fall, in dem die Tiefe kleiner als ein Tiefenschwellenwert ist, die Bestimmungseinheit bestimmt, dass die Punktion möglich ist.

7. Informationsverarbeitungsvorrichtung nach einem der Ansprüche 2 bis 6, wobei:
die Detektionseinheit ferner einen Blutgefäßdurchmesser, der eine Dicke des Blutgefäßes angibt, detektiert, und
die Bestimmungseinheit auf der Grundlage des Blutgefäßdurchmessers bestimmt, ob die Punktion möglich oder unmöglich ist.

8. Informationsverarbeitungsvorrichtung nach Anspruch 7, wobei in einem Fall, in dem der Blutgefäßdurchmesser größer als ein Blutgefäßdurchmesser-Schwellenwert ist, die Bestimmungseinheit bestimmt, dass die Punktion möglich ist.

9. Informationsverarbeitungsvorrichtung nach einem der Ansprüche 2 bis 8, wobei die Detektionseinheit ferner ein Gewebe, das das Blutgefäß umgibt, detektiert, und die Bestimmungseinheit auf der Grundlage des Gewebes bestimmt, ob die Punktion möglich oder unmöglich ist.

10. Informationsverarbeitungsvorrichtung nach Anspruch 9, wobei in einem Fall, in dem ein Abstand zwischen dem Blutgefäß und dem Gewebe gleich oder größer als ein Abstandsschwellenwert ist, die Bestimmungseinheit bestimmt, dass die Punktion möglich ist.

11. Informationsverarbeitungsvorrichtung nach Anspruch 9 oder 10, wobei das Blutgefäß ein Zielblutgefäß ist, das ein Ziel für die Punktion ist, und das Gewebe ein Nichtzielblutgefäß enthält, das nicht das Ziel für die Punktion ist.

12. Informationsverarbeitungsvorrichtung nach einem der Ansprüche 2 bis 11,
wobei die Schwierigkeitsgradinformationen Informationen sind, bei denen sich eine Anzeigeform in Abhängigkeit davon, ob die Punktion möglich oder unmöglich ist, unterscheidet.

13. Informationsverarbeitungsvorrichtung nach einem der Ansprüche 2 bis 12, wobei in einem Fall, in dem eine Bildqualität des Ultraschallbildes höher als eine vorbestimmte Referenz ist, die Bestimmungseinheit das erfasste Fähigkeitsniveau erhöht und bestimmt, ob die Punktion auf der Grundlage einer Blutgefäßbedingung gemäß dem erhöhten Fähigkeitsniveau möglich oder unmöglich ist.

14. Informationsverarbeitungsvorrichtung nach einem der Ansprüche 1 bis 13, wobei die Schwierigkeitsgradinformations-Ausgabeeinheit das Ultraschallbild, zu dem die Schwierigkeitsgradinformationen hinzugefügt wurden, ausgibt.

15. Ultraschalldiagnosevorrichtung, umfassend:
eine Ultraschallsonde, die ein Ultraschallecho von übertragenem Ultraschall empfängt und ein Empfangssignal auf der Grundlage des empfangenen Ultraschallechos ausgibt; eine Bilderzeugungseinheit, die ein Ultraschallbild auf der Grundlage des Empfangssignals, das von der Ultraschallsonde eingegeben wird, erzeugt; und
Informationsverarbeitungsvorrichtung nach einem der Ansprüche 1 bis 14.

16. Informationsverarbeitungsverfahren, das von einem Computer ausgeführt wird, wobei das Verfahren umfasst:
Erfassen eines Ultraschallbildes eines Gewebes einschließlich eines Blutgefäßes einer Untersuchungsperson;
Detektieren des Blutgefäßes aus dem erfassten Ultraschallbild; und
**dadurch gekennzeichnet, dass**:
Ausgeben von Schwierigkeitsgradinformationen, die sich auf einen Schwierigkeitsgrad einer Punktion an dem detektierten Blutgefäß beziehen, gemäß einem Fähigkeitsniveau eines Benutzers, der die Punktion durchführt.

17. Informationsverarbeitungsprogramm, das einen Computer veranlasst, einen Prozess auszuführen, der umfasst:
Erfassen eines Ultraschallbildes eines Gewebes einschließlich eines Blutgefäßes einer Untersuchungsperson;
Detektieren des Blutgefäßes aus dem erfassten Ultraschallbild; und
**dadurch gekennzeichnet, dass**:
Ausgeben von Schwierigkeitsgradinformationen, die sich auf einen Schwierigkeitsgrad einer Punktion an dem detektierten Blutgefäß beziehen, gemäß einem Fähigkeitsniveau eines Benutzers, der die Punktion durchführt.

## Revendications

1. Appareil de traitement d'informations comprenant :
une unité d'acquisition qui acquiert une image ultrasonore d'un tissu incluant un vaisseau sanguin d'un sujet ;
une unité de détection qui détecte le vaisseau sanguin à partir de l'image ultrasonore acquise ; et
**caractérisé en ce qu'**il comprend en outre :
une unité de sortie d'informations de degré de difficulté qui sort des informations de degré de difficulté liées à un degré de difficulté d'une ponction dans le vaisseau sanguin détecté en fonction d'un niveau de compétence d'un utilisateur qui effectue la ponction.

2. Appareil de traitement d'informations selon la revendication 1, comprenant en outre :
une unité de détermination qui détermine si la ponction est possible ou impossible sur la base d'un résultat de détection de l'unité de détection,
dans lequel l'unité de sortie d'informations de degré de difficulté utilise un résultat de détermination de l'unité de détermination comme informations de degré de difficulté.

3. Appareil de traitement d'informations selon la revendication 2, dans lequel l'unité de détermination détermine si la ponction est possible ou impossible sur la base d'une condition de vaisseau sanguin liée à la possibilité ou à l'impossibilité de la ponction en fonction du niveau de compétence de l'utilisateur et du résultat de détection.

4. Appareil de traitement d'informations selon la revendication 2 ou la revendication 3, dans lequel, dans un cas où une qualité d'image de l'image ultrasonore est supérieure à une qualité d'image prédéterminée, l'unité de détermination assouplit la condition de vaisseau sanguin dans laquelle la ponction est possible.

5. Appareil de traitement d'informations selon l'une quelconque des revendications 2 à 4, dans lequel :
l'unité de détection détecte en outre une profondeur depuis une surface corporelle du sujet jusqu'au vaisseau sanguin, et
l'unité de détermination détermine si la ponction est possible ou impossible sur la base de la profondeur.

6. Appareil de traitement d'informations selon la revendication 5, dans lequel, dans un cas où la profondeur est inférieure à une valeur seuil de profondeur, l'unité de détermination détermine que la ponction est possible.

7. Appareil de traitement d'informations selon l'une quelconque des revendications 2 à 6, dans lequel :
l'unité de détection détecte en outre un diamètre de vaisseau sanguin indiquant une épaisseur du vaisseau sanguin, et
l'unité de détermination détermine si la ponction est possible ou impossible sur la base du diamètre de vaisseau sanguin.

8. Appareil de traitement d'informations selon la revendication 7, dans lequel, dans un cas où le diamètre de vaisseau sanguin est supérieur à une valeur seuil de diamètre de vaisseau sanguin, l'unité de détermination détermine que la ponction est possible.

9. Appareil de traitement d'informations selon l'une quelconque des revendications 2 à 8, dans lequel
l'unité de détection détecte en outre un tissu entourant le vaisseau sanguin, et
l'unité de détermination détermine si la ponction est possible ou impossible sur la base du tissu.

10. Appareil de traitement d'informations selon la revendication 9, dans lequel, dans un cas où une distance entre le vaisseau sanguin et le tissu est égale ou supérieure à une valeur seuil d'espacement, l'unité de détermination détermine que la ponction est possible.

11. Appareil de traitement d'informations selon la revendication 9 ou la revendication 10, dans lequel le vaisseau sanguin est un vaisseau sanguin cible qui est une cible pour la ponction, et le tissu inclut un vaisseau sanguin non ciblé qui n'est pas la cible pour la ponction.

12. Appareil de traitement d'informations selon l'une quelconque des revendications 2 à 11,
dans lequel les informations de degré de difficulté sont des informations dont une forme d'affichage diffère selon que la ponction est possible ou impossible.

13. Appareil de traitement d'informations selon l'une quelconque des revendications 2 à 12, dans lequel, dans un cas où une qualité d'image de l'image ultrasonore est supérieure à une référence prédéterminée, l'unité de détermination augmente le niveau de compétence, qui est acquis, et détermine si la ponction est possible ou impossible sur la base d'une condition de vaisseau sanguin en fonction du niveau de compétence augmenté.

14. Appareil de traitement d'informations selon l'une quelconque des revendications 1 à 13, dans lequel l'unité de sortie d'informations de degré de difficulté sort l'image ultrasonore à laquelle les informations de degré de difficulté sont ajoutées.

15. Appareil de diagnostic par ultrasons comprenant :
une sonde ultrasonore qui reçoit un écho ultrasonore provenant des ultrasons transmis et sort un signal de réception sur la base de l'écho ultrasonore reçu ;
une unité de génération d'images qui génère une image ultrasonore sur la base du signal de réception entrant provenant de la sonde ultrasonore ; et
l'appareil de traitement d'informations selon l'une quelconque des revendications 1 à 14.

16. Procédé de traitement d'informations exécuté par un ordinateur, le procédé comprenant :
acquérir une image ultrasonore d'un tissu incluant un vaisseau sanguin d'un sujet ;
détecter le vaisseau sanguin à partir de l'image ultrasonore acquise ; et
**caractérisé par** :
sortir des informations de degré de difficulté liées à un degré de difficulté d'une ponction dans le vaisseau sanguin détecté en fonction d'un niveau de compétence d'un utilisateur qui effectue la ponction.

17. Programme de traitement d'informations amenant un ordinateur à exécuter un processus comprenant :
acquérir une image ultrasonore d'un tissu incluant un vaisseau sanguin d'un sujet ;
détecter le vaisseau sanguin à partir de l'image ultrasonore acquise ; et
**caractérisé par** :
sortir des informations de degré de difficulté liées à un degré de difficulté d'une ponction dans le vaisseau sanguin détecté en fonction d'un niveau de compétence d'un utilisateur qui effectue la ponction.
